(19) Europäisches Patentamt
European Patent Office
Office européen des brevets

(11) Numéro de publication : 0 605 295 A1

(12) DEMANDE DE BREVET EUROPEEN

(21) Numéro de dépôt : 93403137.8

(22) Date de dépôt : 22.12.93

(51) Int. Cl.[5] : C07D 513/04, A61K 31/425, // (C07D513/04, 277:00, 235:00)

(30) Priorité : 30.12.92 FR 9215889

(43) Date de publication de la demande : 06.07.94 Bulletin 94/27

(84) Etats contractants désignés : AT BE CH DE DK ES FR GB GR IE IT LI LU MC NL PT SE

(71) Demandeur : SYNTHELABO
22, Avenue Galilée
F-92350 Le Plessis Robinson (FR)

(72) Inventeur : Sevrin, Mireille
73, rue Raymond Losserand
F-75014 Paris (FR)
Inventeur : Morel, Claude
25, rue Gabriel Péri
F-78114 Magny les Hameaux (FR)
Inventeur : Mangane, Michel
44, avenue Marcel Cachin
F-92320 Chatillon s/Bagneux (FR)
Inventeur : George, Pascal
19, rue des Quatre Vents
F-78730 St Arnoult en Yvelines (FR)

(74) Mandataire : Ludwig, Jacques et al
SYNTHELABO,
Service Brevets,
B.P. 72
F-92352 Le Plessis Robinson Cédex (FR)

(54) **Dérivés d'acide 2-thiénylimidazo 2,1-b benzothiazole-3-acétique, leur préparation et leur application en thérapeutique.**

(57) Composés répondant à la formule générale (I)

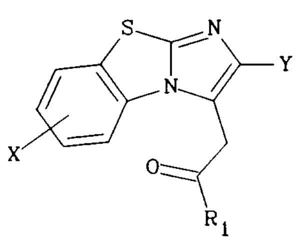

(I)

dans laquelle
$R_1$ représente un groupe hydroxy, un groupe alcoxy en $C_1$-$C_4$ ou un groupe amino de formule générale $-NR_4R_5$ dans laquelle $R_4$ et $R_5$ représentent chacun, indépendamment l'un de l'autre, un atome d'hydrogène, un groupe alkyle en $C_1$-$C_4$ droit ou ramifié, un groupe allyle ou un groupe méthoxyéthyle, ou bien encore $-NR_4R_5$ représente un hétérocycle comportant de 3 à 6 atomes de carbone,
Y représente un groupe thiényle éventuellement substitué par un groupe alkyle, et
X représente un atome d'hydrogène ou d'halogène.
Application en thérapeutique.

EP 0 605 295 A1

La présente invention a pour objet des dérivés d'acide 2-thiénylimidazo[2,1-*b*]benzothiazole-3-acétique, leur préparation et leur application en thérapeutique.

Les composés de l'invention répondent à la formule générale (I)

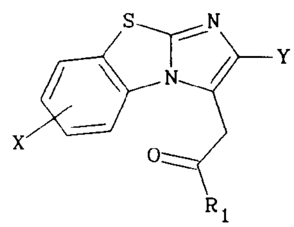

(I)

dans laquelle

$R_1$ représente un groupe hydroxy, un groupe alcoxy en $C_1$-$C_4$ ou un groupe amino de formule générale $-NR_4R_5$ dans laquelle $R_4$ et $R_5$ représentent chacun, indépendamment l'un de l'autre, un atome d'hydrogène, un groupe alkyle en $C_1$-$C_4$ droit ou ramifié, un groupe allyle ou un groupe méthoxyéthyle, ou bien encore $-NR_4R_5$ représente un hétérocycle comportant de 3 à 6 atomes de carbone,

X représente un atome d'hydrogène ou d'halogène, et

Y représente un groupe thiényle de formule générale (IA) ou (IB)

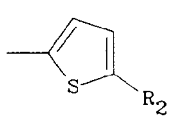

(IA)

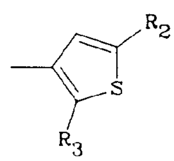

(IB)

dans lesquelles $R_2$ et $R_3$ représentent chacun, indépendamment l'un de l'autre, un groupe alkyle en $C_1$-$C_4$ droit ou ramifié.

Les composés de l'invention peuvent se présenter à l'état de bases libres ou de sels d'addition à des acides.

$R_1$ représente, de préférence, un groupe méthylamino.

Conformément à l'invention, on peut préparer ces composés selon un procédé illustré dans le schéma ci-après.

On fait réagir un 2-thiénylimidazo[2,1-*b*]benzothiazole de formule générale (II), dans laquelle X et Y sont tels que

## Schéma

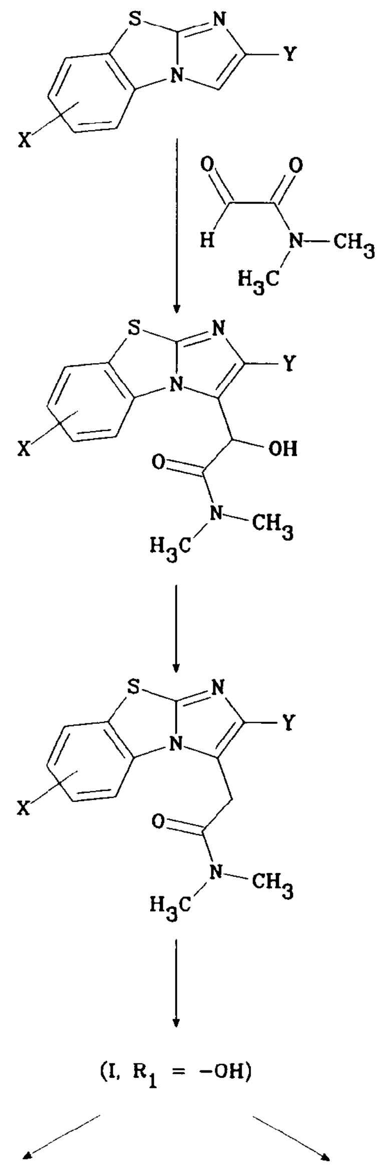

(II)

(III)

(IV)

(I, $R_1$ = -OH)

(I, $R_1$ = -O-alkyle)

(I, $R_1$ = $-NR_4R_5$)

définis ci-dessus, avec le *N,N*-diméthylglyoxamide (que l'on prépare *in situ* au moyen de 2,2-diéthoxy-*N,N*-diméthylacétamide, comme décrit dans la demande de brevet EP-251859) dans un solvant protique tel que l'acide acétique à une température de 20 à 80°C.

On traite ensuite le dérivé d'α-hydroxyacétamide de formule générale (III) avec un polyhalogénure d'acide sulfurique ou phosphorique, par exemple le chlorure de thionyle ou l'oxychlorure de phosphore, ou tout autre agent équivalent, dans un solvant inerte, par exemple un solvant chloré ou éthéré tel que le dichlorométhane ou le tétrahydrofurane, à une température de 20 à 80°C, pour former le dérivé de α-halogénoacétamide correspondant, puis on fait réagir ce dernier soit avec un agent réducteur tel qu'un hydrure alcalin simple ou complexe, par exemple le borohydrure de sodium ou de potassium, dans un solvant protique, par exemple un alcool aliphatique tel que le méthanol ou l'éthanol, ou dans un solvant inerte miscible à l'eau, par exemple le dioxane ou le tétrahydrofurane, à une température de -40 à 40°C, soit avec un agent réducteur tel qu'un hyposulfite ou un dithionite alcalin, par exemple l'hyposulfite ou le dithionite de sodium, ou encore avec l'hydroxyméthylsulfoxylate de sodium (Rongalite®), dans un solvant inerte, par exemple un solvant chloré tel que le dichlorométhane, éventuellement en présence d'un cosolvant inerte miscible à l'eau, par exemple le *N,N*- diméthylformamide ou la *N*-méthylpyrrolidone, à une température de 20 à 40°C.

On obtient ainsi un 2-thiénylimidazo[2,1-*b*]benzothiazole-3-acétamide de formule générale (IV), qui correspond à la formule générale (I) lorsque $R_1$ représente un groupe de formule $-N(CH_3)_2$.

Si on le désire, on transforme ce composé en l'acide de formule générale (I) dans laquelle $R_1$ représente un groupe hydroxy, par hydrolyse au moyen d'une base forte, par exemple l'hydroxyde de sodium, dans un solvant protique, par exemple le 2-méthoxyéthanol, en présence d'eau.

Si on désire préparer un composé de formule générale (I) dans laquelle $R_1$ représente un groupe de formule générale $-NR_4R_5$ autre qu'un groupe de formule $-N(CH_3)_2$, on fait réagir l'acide de formule générale (I, $R_1$ = OH) avec le *N,N'*-carbonyldiimidazole, dans un solvant inerte, par exemple un solvant chloré ou éthéré tel que le dichlorométhane ou le tétrahydrofurane, à une température de 20 à 50°C, pour obtenir l'imidazolide correspondant, et finalement on traite ce dernier avec une amine de formule générale $HNR_4R_5$ (dans laquelle $R_4$ et $R_5$ sont tels que définis ci-dessus) à une température de 0 à 25°C.

Enfin, si on le désire, on peut transformer l'acide de formule générale (I, $R_1$ = OH) en ester (I, $R_1$ = -O-alkyle) selon des méthodes connues.

Les 2-thiénylimidazo[2,1-*b*]benzothiazoles de départ, de formule générale (II), peuvent être préparés par des méthodes analogues à celle décrite dans *Khim. Geterotsikl. Soedin.*, **9**, 1271-1274 (1972).

Les exemples qui vont suivre illustrent la préparation de quelques composés de l'invention. Les microanalyses élémentaires, et les spectres I.R. et R.M.N. confirment les structures des composés obtenus.
Les numéros indiqués entre parenthèses dans les titres des exemples correspondent à ceux de la lère colonne du tableau donné plus loin.

Exemple 1 (Composé N°5)

*N,N*-Diméthyl-2-(5-méthylthién-2-yl)imidazo[2,1-*b*]benzothiazole-3-acétamide.

1.1. α-Hydroxy-*N,N*-diméthyl-2-(5-méthylthién-2-yl)imidazo[2,1-*b*]benzothiazole-3-acétamide.

Dans un ballon de 1000 ml on mélange 30 g (0,171 mole) de 2,2-diéthoxy-*N,N*-diméthylacétamide et 460 ml d'acide acétique, on chauffe la solution à 45-50°C et on ajoute, goutte à goutte, 4,6 ml d'acide chlorhydrique à 36% (soit 1,65 g ou 0,045 équivalent).

On agite le mélange pendant 1h à 45-50°C.

On ajoute ensuite 14 g (0,171 mole) d'acétate de sodium, on agite pendant 15 minutes à 45-50°C puis, en une seule fois, on ajoute 15,4 g (0,057 mole) de 2-(5-méthylthién-2-yl)imidazo[2,1-*b*]benzothiazole, et on chauffe le mélange en l'agitant à 50-55°C pendant 3h.

On évapore le solvant sous pression réduite, on reprend le solide avec de l'eau et du dichlorométhane, on élimine un résidu par filtration, on sépare la phase organique, on la lave à l'eau bicarbonatée et on la sèche sur sulfate de sodium.

On évapore le solvant sous pression réduite et on purifie le résidu par chromatographie sur colonne de gel de silice en éluant avec un mélange 98/2 de dichlorométhane/méthanol. On obtient 15,6 g de composé qu'on utilise tel quel dans l'étape suivante.

1.2. Chlorhydrate de α-chloro-*N,N*-diméthyl-2-(5-méthylthién-2-yl)imidazo[2,1-*b*]benzothiazole-3-acétamide.

On traite 15,6 g (0,0474 mole) de α-hydroxy-*N,N*-diméthyl-2-(5-méthylthién-2-yl)imidazo[2,1-*b*]benzothiazole-3-acétamide avec 34,8 ml (ou 57,1 g, soit 0,48 mole) de chlorure de thionyle dans 200 ml de dichlorométhane.

On chauffe le mélange au reflux pendant 5h, on évapore le solvant et l'excès de chlorure de thionyle sous pression réduite, et on fait deux entrainements au toluène sous pression réduite. On isole le solide obtenu que l'on sèche sous vide en présence de potasse en pastilles.

On obtient 17,8 g d'un solide marron qu'on utilise tel quel dans l'étape suivante.

1.3. *N,N*-Diméthyl-2-(5-méthylthién-2-yl)imidazo[2,1-*b*]benzothiazole-3-acétamide.

On traite 40 g (0,0938 mole) de chlorhydrate de α-chloro-*N,N*-diméthyl-2-(5-méthylthién-2-yl)imidazo[2,1-*b*]benzothia- zole-3-acétamide en solution dans 1200 ml de dichlorométhane par 43,6 g (0,283 mole) de Rongalite®, en agitant le mélange à la température ambiante pendant 6h.

On filtre la suspension, on rince avec du dichlorométhane et on lave la phase organique (pH = 1) avec 500 ml de soude 0,5N jusqu'à pH > 10, puis à la neutralité avec une solution saturée de chlorure de sodium.

On sèche la phase organique sur sulfate de sodium et on évapore le solvant sous pression réduite.

On obtient un solide de couleur beige qu'on purifie par chromatographie sur colonne de gel de silice en éluant avec un mélange 95/5 de dichlorométhane/méthanol.

On obtient 23,9 g d'un solide blanc.

Point de fusion : 216-218°C

Exemple 2 (Composé N°11)

Acide 2-(5-méthylthién-2-yl)imidazo[2,1-*b*]benzothiazole-3-acétique.

Dans un ballon de 1 litre on introduit 23,8 g (0,067 mole) de *N,N*-diméthyl-2-(5-méthylthién-2-yl)imidazo[2,1-*b*]benzothiazole-3-acétamide et 450 ml de 2-méthoxyéthanol, on agite le mélange et on le chauffe au bain d'huile vers 130°C jusqu'à dissolution du solide.

On ajoute à chaud, avec précautions, une solution de 13,4 g (0,335 mole) de soude en pastilles dans 70 ml d'eau puis, après 30 minutes, de nouveau 13,4g (0,335 mole) de soude en pastilles dans 70 ml d'eau, et on maintient le chauffage pendant 7h30, en suivant l'avancement de la réaction par chromatographie sur couche mince.

On refroidit le mélange vers 20°C, on évapore le solvant sous pression réduite, sans dépasser 60°C.

On ajoute au résidu 1200 ml d'eau, on élimine un insoluble par filtration, on ajoute au filtrat 150 ml d'acide acétique jusqu'à un pH = 4. On obtient un précipité de couleur jaune que l'on agite pendant 30 minutes en refroidissant vers 5°C.

On sépare le solide par filtration, on le lave à l'eau puis à l'éther diéthylique, et on le sèche sous vide vers 60°C.

On obtient 17,5 g de composé dont on purifie 1,65 g (0,005 mole) par recristallisation dans 250 ml de méthanol à ébullition.

On isole 1,1 g d'un solide de couleur jaune clair.

Point de fusion : 255-259°C

Exemple 3 (Composé N°1)

2-(5-Méthylthién-2-yl)imidazo[2,1-*b*]benzothiazole-3-acétamide.

On traite à la température ambiante 1,6 g (0,005 mole) d'acide 2-(5-méthylthién-2-yl)imidazo[2,1-*b*]benzothiazole-3-acétique avec 0,85 g (0,005 mole) de *N,N'*-carbonyldiimidazole dans 30 ml de tétrahydrofurane anhydre pendant 3h30.

On refroidit le mélange par un bain de glace, on ajoute un excès d'ammoniac dans 10 ml de tétrahydrofurane anhydre refroidi à -10°C, et on agite le mélange pendant 4h à la température ambiante.

On évapore le solvant sous pression réduite, on collecte le solide par filtration, on le lave à l'eau jusqu'à neutralité, puis à l'éthanol, et enfin à l'éther éthylique. Après recristallisation dans le méthanol on obtient 1 g de composé beige.

Point de fusion : 243-245°C.

Exemple 4 (Composé N°4)

2-(5-Méthylthién-2-yl)-*N*-propylimidazo[2,1-*b*]benzothiazole-3-acétamide.

On traite à la température ambiante 1,6 g (0,005 mole) d'acide 2-(5-méthylthién-2-yl)imidazo[2,1-*b*]benzothiazole-3-acétique avec 0,85 g (0,005 mole) de *N,N'*-carbonyldiimidazole dans 30 ml de tétrahydrofurane anhydre pendant 3h30.

On ajoute une solution composée de 3 g (0,05 mole) de propylamine dans 10 ml de tétrahydrofurane anhydre, et on agite le mélange pendant 4h à la température ambiante.

On évapore le solvant sous pression réduite, on collecte le composé par filtration, on le lave à l'eau jusqu'à neutralité, puis à l'éthanol et enfin à l'éther éthylique, et on le purifie par recristallisation dans le méthanol.

On obtient 1,1 g de solide jaune clair.

Point de fusion : 204-206°C.

Exemple 5 (Composé N°12)

2-(5-Méthylthién-2-yl)imidazo[2,1-*b*]benzothiazole-3-acétate d'éthyle.

A partir de 1,65 g (0,005 mole) d'acide 2-(5-méthylthién-2-yl)imidazo[2,1-*b*]benzothiazole-3-acétique on prépare l'ester éthylique par reflux pendant 6h dans l'éthanol en présence d'acide sulfurique.

Après chromatographie sur colonne de gel de silice, en éluant avec un mélange 95/5 de dichlorométhane/méthanol, et après recristallisation dans l'acétate d'éthyle, on obtient un solide de couleur beige.

Point de fusion : 124-126°C.

Exemple 6 (Composé N°18)

*N,N*-Diméthyl-2-(2,5-diméthylthién-3-yl)imidazo[2,1-*b*]benzothiazole-3-acétamide.

6.1. α-Hydroxy-*N,N*-diméthyl-2-(2,5-diméthylthién-3-yl)imidazo[2,1-*b*]benzothiazole-3-acétamide.

Dans un ballon de 1000 ml on mélange 40,7 g (0,232 mole) de 2,2-diéthoxy-*N,N*-diméthylacétamide et 630 ml d'acide acétique, on chauffe la solution à 50°C, on ajoute, goutte à goutte, 6,3 ml (0,06 mole) d'acide chlorhydrique à 36%, et on poursuit l'agitation à 50°C pendant 1h.

On ajoute ensuite 19 g (0,232 mole) d'acétate de sodium, on agite pendant 15 minutes, puis on ajoute, en une seule fois, 22 g (0,077 mole) de 2-(2,5-diméthylthién-3-yl)imidazo[2,1-*b*]benzothiazole, et on maintient le chauffage à 50°C pendant 6h.

On évapore le solvant sous pression réduite et à une température inférieure à 50°C, on reprend le résidu avec du dichlorométhane et de l'eau, on ajuste le pH à 9 avec de l'ammoniaque, on sépare la phase organique, on la lave à l'eau, on la sèche sur sulfate de sodium et on évapore le solvant sous pression réduite.

On obtient 31 g de produit huileux qu'on utilise tel quel dans l'étape suivante.

6.2. Chlorhydrate de α-chloro-*N,N*-diméthyl-2-(2,5-diméthylthién-3-yl)imidazo[2,1-*b*]benzothiazole-3-acétamide.

Dans un ballon de 500 ml on introduit 31 g (0,077 mole) de α-hydroxy-*N,N*-diméthyl-2-(2,5-diméthylthién-3-yl)imidazo[2,1-*b*]benzothiazole-3-acétamide, 150 ml de dichlorométhane et 2 ml de *N,N*-diméthylformamide, on ajoute une solution de 58 ml (0,77 mole) de chlorure de thionyle dans 100 ml de dichlorométhane, et on chauffe le mélange au reflux pendant 6h.

On évapore le solvant et l'excès de chlorure de thionyle sous pression réduite, on fait deux entraînements au toluène sous pression réduite, on évapore les solvants sous vide poussé jusqu'à poids constant, et on obtient 34 g d'une huile qui cristallise.

6.3. *N,N*-Diméthyl-2-(2,5-diméthylthién-3-yl)imidazo[2,1-*b*]benzothiazole-3-acétamide.

On traite 34 g (0,077 mole) de chlorhydrate de α-chloro-*N,N*-diméthyl-2-(2,5-diméthylthién-3-yl)imidazo[2,1-*b*]benzothiazole-3-acétamide en solution dans 500 ml de dichlorométhane avec 36,9 g (0,24 mole) de Rongalite®, en agitant le mélange à température ambiante pendant 24h.

On sépare la phase organique par décantation, on lave le résidu solide avec du dichlorométhane, on lave la phase organique avec une solution saturée d'hydrogénocarbonate de sodium puis avec de l'eau, on la sèche sur sulfate de sodium, on évapore le solvant sous pression réduite et on obtient 22 g de solide.

Après recristallisation dans l'éthanol et traitement au noir végétal on obtient finalement 17 g de cristaux jaune pâle.

Point de fusion : 194-195°C.

Exemple 7 (Composé N°23)

Acide 2-(2,5-diméthylthién-3-yl)imidazo[2,1-*b*]benzothiazole-3-acétique.

Dans un ballon de 1000 ml on introduit 14 g (0,0379 mole) de *N,N*-diméthyl-2-(2,5-diméthylthién-3-yl)imidazo[2,1-*b*]benzothiazole-3-acétamide et 350 ml de 2-méthoxyéthanol, on chauffe la solution à 100°C, on ajoute 25 ml de solution à 30% d'hydroxyde de sodium (0,189 mole), on chauffe à l'ébullition pendant 30 minutes, on ajoute encore 25 ml d'hydroxyde de sodium à 30%, et on maintient le chauffage pendant 4h.

On ajoute de nouveau 25 ml de solution à 30% d'hydroxyde de sodium, et on chauffe encore pendant 3h.

On évapore les solvants sous pression réduite, on reprend le résidu avec 500 ml d'eau, on sépare l'insoluble par filtration, et on ajuste le pH du filtrat à 4 avec de l'acide acétique. On obtient un précipité jaune qu'on sépare par filtration, et on le lave avec de l'eau puis avec de l'éther diéthylique.

Après cristallisation dans le méthanol on obtient 9 g de cristaux jaune clair.

Point de fusion : 249-250°C.

Exemple 8 (Composé N°14)

2-(2,5-Diméthylthién-3-yl)-*N*-méthylimidazo[2,1-*b*]benzothiazole-3-acétamide.

On traite à température ambiante une solution de 1,5 g (0,0044 mole) d'acide 2-(2,5-diméthylthién-3-yl)imidazo[2,1-*b*]benzothiazole-3-acétique dans 30 ml de tétrahydrofurane anhydre avec 0,85 g (0,005 mole) de *N,N'*-carbonyldiimidazole pendant 30 minutes.

On refroidit à 0°C la solution devenue limpide, et on y fait passer un courant de méthylamine pendant 30 minutes.

On laisse le mélange revenir à température ambiante, on poursuit l'agitation pendant 3h, un évapore le solvant sous pression réduite, on reprend le résidu avec 10 ml d'eau, on l'extrait avec du dichlorométhane, on sépare

la phase organique, on la lave à l'eau, on la sèche sur sulfate de sodium, on évapore le solvant sous pression réduite, et on fait cristalliser le résidu dans l'acétate d'éthyle.
On obtient finalement 1 g de cristaux jaune pâle.
Point de fusion : 238-239°C.

Exemple 9 (Composé N°21)

6-Chloro-*N,N*-diméthyl-2-(2,5-diméthylthién-3-yl)imidazo[2,1-*b*]benzothiazole-3-acétamide.

9.1. 6-Chloro-α-hydroxy-*N,N*-diméthyl-2-(2,5-diméthylthién-3-yl)imidazo[2,1-*b*]benzothiazole-3-acétamide.

On chauffe un mélange de 27 g (0,153 mole) de 2,2-diéthoxy-*N,N*-diméthylacétamide, 4 ml (0,04 mole) d'acide chlorhydrique à 36% et 200 ml d'acide acétique pendant 1h à 45°C.

On ajoute 12,56 g (0,153 mole) d'acétate de sodium, on poursuit le chauffage pendant 15 minutes, on ajoute 15 g (0,0479 mole) de 6-chloro-2-(2,5-diméthylthién-3-yl)imidazo[2,1-*b*]benzothiazole, et on chauffe le mélange à 50°C pendant 6h.

On élimine l'acide acétique par distillation sous pression réduite, on reprend le résidu avec un mélange d'eau et de dichlorométhane, on ajoute de l'ammoniaque, on sépare la phase organique, on la lave, on la sèche sur sulfate de sodium, et on évapore le solvant sous pression réduite.

On obtient 18 g de produit huileux qui cristallise.

9.2. 6-Chloro-*N,N*-diméthyl-2-(2,5-diméthylthién-3-yl)imidazo[2,1-*b*]benzothiazole-3-acétamide.

On traite 18 g (0,0431 mole) de 6-chloro-α-hydroxy-*N,N*-diméthyl-2-(2,5-diméthylthién-3-yl)imidazo[2,1-*b*]benzothiazole-3-acétamide avec 31 ml (0,43 mole) de chlorure de thionyle, 2 ml de *N,N*-diméthylformamide et 100 ml de dichlorométhane à la température du reflux pendant 6h.

On élimine le solvant et l'excès de chlorure de thionyle sous pression réduite, on reprend le résidu avec de l'éther diéthylique, on lave le solide avec de l'éther diéthylique, et on le sèche.

On obtient 21 g de chlorhydrate de α,6-dichloro-*N,N*-diméthyl-2-(2,5-diméthylthién-3-yl)imidazo[2,1-*b*]benzothiazole-3-acétamide qu'on réduit avec 21 g (0,136 mole) de Rongalite® dans 300 ml de dichlorométhane à température ambiante pendant 24h.

On sépare la solution par décantation, on lave le solide avec du dichlorométhane, on lave la phase organique avec une solution saturée d'hydrogénocarbonate de sodium puis avec de l'eau, on la sèche sur sulfate de sodium, et on évapore le solvant sous pression réduite.

On obtient une huile qui cristallise, et qu'on recristallise dans l'acétonitrile.

On isole finalement 14,4 g d'aiguilles jaunes.

Point de fusion : 202-203°C.

Le tableau qui suit illustre les structures chimiques et les propriétés physiques de quelques composés selon l'invention.
Tous les composés sont à l'état de bases.

Tableau

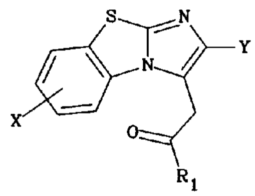

(I)

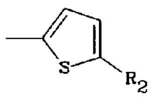

(IA)

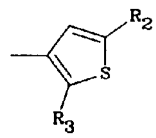

(IB)

| N° | $R_1$ | Y | $R_2$ | $R_3$ | X | F (°C) |
|---|---|---|---|---|---|---|
| 1 | $-NH_2$ | IA | $-CH_3$ | - | H | 243-245 |
| 2 | $-NH-CH_3$ | IA | $-CH_3$ | - | H | 271-273 |
| 3 | $-NH-CH_2CH_3$ | IA | $-CH_3$ | - | H | 246-248 |
| 4 | $-NH-CH_2CH_2CH_3$ | IA | $-CH_3$ | - | H | 204-206 |
| 5 | $-N(CH_3)_2$ | IA | $-CH_3$ | - | H | 216-218 |
| 6 | $-N(CH_3)-CH_2CH_2CH_3$ | IA | $-CH_3$ | - | H | 154-156 |
| 7 | $-N(CH_3)-CH_2CH=CH_2$ | IA | $-CH_3$ | - | H | 146-148 |
| 8 | $-N(CH_2CH_3)-CH_2CH_2CH_3$ | IA | $-CH_3$ | - | H | 125-127 |
| 9 | $-N(CH_2CH_2CH_3)_2$ | IA | $-CH_3$ | - | H | 153-155 |
| 10 | 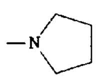 | IA | $-CH_3$ | - | H | 262-264 |
| 11 | $-OH$ | IA | $-CH_3$ | - | H | 255-259 |
| 12 | $-O-CH_2CH_3$ | IA | $-CH_3$ | - | H | 124-126 |
| 13 | $-NH_2$ | IB | $-CH_3$ | $-CH_3$ | H | 258-259 |
| 14 | $-NH-CH_3$ | IB | $-CH_3$ | $-CH_3$ | H | 238-239 |
| 15 | $-NH-CH_2CH_3$ | IB | $-CH_3$ | $-CH_3$ | H | 162-163 |
| 16 | $-NH-CH_2CH_2CH_3$ | IB | $-CH_3$ | $-CH_3$ | H | 176-177 |
| 17 | $-NH-CH_2CH_2-O-CH_3$ | IB | $-CH_3$ | $-CH_3$ | H | 126-127 |

| N° | $R_1$ | Y | $R_2$ | $R_3$ | X | F (°C) |
|---|---|---|---|---|---|---|
| 18 | $-N(CH_3)_2$ | IB | $-CH_3$ | $-CH_3$ | H | 194-195 |
| 19 | $-NH-CH_3$ | IB | $-CH_3$ | $-CH_3$ | 6-Cl | 233-234 |
| 20 | $-NH-CH_2CH_2CH_3$ | IB | $-CH_3$ | $-CH_3$ | 6-Cl | 217-218 |
| 21 | $-N(CH_3)_2$ | IB | $-CH_3$ | $-CH_3$ | 6-Cl | 202-203 |
| 22 | $-N(CH_3)_2$ | IB | $-CH_3$ | $-CH_2CH_2CH_3$ | H | 194-195 |
| 23 | -OH | IB | $-CH_3$ | $-CH_3$ | H | 249-250 |
| 24 | -OH | IB | $-CH_3$ | $-CH_3$ | 6-Cl | >260 |
| 25 | 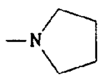 | IB | $-CH_3$ | $-CH_3$ | H | 175-176 |

Les composés de l'invention ont été soumis à des essais pharmacologiques qui ont mis en évidence leur intérêt comme substances à activités thérapeutiques.

Etude des liaisons membranaires vis-à-vis des récepteurs $\omega_1$ (benzodiazépiniques de type 1) et $\omega_2$ (benzodiazépiniques de type 2).

L'affinité des composés pour les récepteurs $\omega_1$ du cervelet et $\omega_2$ de la moelle épinière a été déterminée selon une variante de la méthode décrite par S. Z. Langer et S. Arbilla dans *Fund. Clin. Pharmacol.*, **2**, 159-170 (1988), avec utilisation de $^3$H-flumazenil au lieu de $^3$H-diazepam comme radioligand. On homogénéise le tissu du cervelet ou de la moelle épinière pendant 60 s dans 120 ou 30 volumes, respectivement, de tampon glacé (50 mM Tris/HCl, pH 7,4, 120 mM NaCl, 5 mM KCl) puis, après dilution à 1/3, on fait incuber la suspension avec du $^3$H-flumazenil (activité spécifique 78 Ci/mmole, New England Nuclear) à une concentration de 1 nM et avec les composés de l'invention à différentes concentrations, dans un volume final de 525 µl. Après 30 minutes d'incubation à 0°C on filtre les échantillons sous vide sur des filtres Whatman GF/B® et on les lave immédiatement avec du tampon glacé. La liaison spécifique du $^3$H-flumazenil est déterminée en présence de diazépam 1 µM non marqué. On analyse les données selon les méthodes usuelles et on calcule la concentration $CI_{50}$, concentration qui inhibe de 50% la liaison du $^3$H-flumazenil.
Les $CI_{50}$ des composés de l'invention se situent, dans ces essais, entre 1 et 1000 nM.

Etude de l'activité anticonvulsivante.

Activité vis-à-vis des convulsions maximales induites chez la souris par électrochoc ou par injection de pentétrazol.

Le protocole de cet essai est décrit par E. A. Swinyard et J. H. Woodhead dans *Antiepileptic Drugs,* Raven Press, New York, 111-126 (1982).
30 minutes après administration intrapéritonéale du composé à tester, on note le nombre de souris présentant des convulsions (extensions des pattes arrière), soit immédiatement après application d'un courant électrique (0,4 s, 60 mA, 50 Hz) à l'aide d'électrodes transcornéennes, soit pendant les 30 minutes qui suivent l'injection sous-cutanée de penté- trazol (125 mg/kg). Les résultats sont exprimés par la $DA_{50}$, dose qui protège 50% des animaux, calculée selon la méthode de J. T. Lichtfield et F. Wilcoxon (*J. Pharm. Exp. Ther.*, **96**, 99-113 (1949)) à partir de 3 ou 4 doses administrées chacune à un groupe de 8 à 10 souris.
Les $DA_{50}$ des composés de l'invention se situent, dans cet essai, entre 1 et 100 mg/kg par la voie intrapéritonéale.

Activité vis-à-vis des convulsions induites chez la souris par l'isoniazide.

L'activité intrinsèque des composés est déterminée par le temps de latence d'apparition des convulsions induites par l'administration sous-cutanée d'isoniazide (800 mg/kg) simultanément avec le composé à tester, injecté par voie intrapéritonéale, selon le protocole décrit par G. Perrault, E. Morel, D. Sanger et B. Zivkovic dans *Eur. J. Pharmacol.,* **156**, 189-196 (1988). Les résultats sont exprimés par la $DA_{50}$, dose qui produit 50% de l'effet maximal, par rapport aux animaux témoins, déterminée à partir de 3 ou 4 doses adminis- trées chacune à un groupe de 8 à 10 souris.
Les $DA_{50}$ des composés de l'invention se situent, dans cet essai, entre 1 et 50 mg/kg par la voie intrapéritonéale et, selon les composés, l'effet maximal peut aller jusqu'à 350%.

Etude de l'activité anxiolytique.

L'activité anxiolytique est évaluée chez le rat dans le test de conflit de prise de boisson, selon la méthode décrite par J. R. Vogel, B. Beer et D. E. Clody dans *Psychopharmacologia* (Berl.), **21**, 1-7 (1971).
Après une diète hydrique de 48h, le rat est placé dans une enceinte isolée du bruit et équipée d'une pipette d'eau reliée à un anxiomètre délivrant un léger choc électrique tous les 20 coups de langue. Le nombre de chocs reçus est automatiquement compté pendant 3 minutes, et permet d'évaluer l'activité anxiolytique des composés testés. Les résultats sont exprimés par la dose efficace minimale (DEM), dose qui produit une augmentation significative du nombre de chocs reçus, par rapport au nombre observé chez les animaux témoins.
Les DEM des composés del'invention se situent, dans cet essai, entre 1 et 100 mg/kg par la voie intrapéritonéale ou orale.

Les résultats des essais effectués sur les composés de l'invention montrent que, *in vitro,* ils déplacent le $^3$H-flumazénil de ses sites de liaison spécifiques au niveau du cervelet et de la moelle épinière ; par conséquent ils présentent une affinité pour les sites $\omega_1$ et $\omega_2$ (benzodiazépiniques de type 1 et de type 2) situés au sein du complexe macromoléculaire $GABA_A$-sites ω-canal chlorure.
*In vivo* ils se comportent comme des agonistes complets ou partiels, ou comme des antagonistes vis-à-vis de ces récepteurs.
Ils possèdent des propriétés anticonvulsivantes et anxiolytiques et, par conséquent, peuvent être utilisés pour le traitement d'affections liées aux désordres de la transmission GABAergique, tels que l'anxiété, les troubles du sommeil, l'épilepsie, la spasticité, les contractures musculaires, les troubles cognitifs, les troubles du sevrage vis-à-vis de l'alcoolisme, etc.

A cet effet ils peuvent être présentés sous toutes formes galéniques, associés à des excipients appropriés, pour l'administration entérale ou parentérale, par exemple sous forme de comprimés, dragées, gélules, capsules, solutions ou suspensions buvables ou injectables, suppositoires, etc, dosés pour permettre une administration journalière de 1 à 1000 mg de substance active.

## Revendications

1. Composé répondant à la formule générale (I)

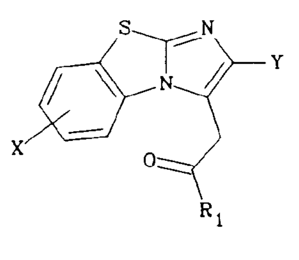

(I)

dans laquelle
$R_1$ représente un groupe hydroxy, un groupe alcoxy en $C_1$-$C_4$ ou un groupe amino de formule générale $-NR_4R_5$ dans laquelle $R_4$ et $R_5$ représentent chacun, indépendamment l'un de l'autre, un atome d'hydrogène, un groupe alkyle en $C_1$-$C_4$ droit ou ramifié, un groupe allyle ou un groupe méthoxyéthyle, ou bien encore $-NR_4R_5$ représente un hétérocycle comportant de 3 à 6 atomes de carbone,
X représente un atome d'hydrogène ou d'halogène, et
Y représente un groupe thiényle de formule générale (IA) ou (IB)

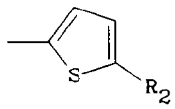 (IA) 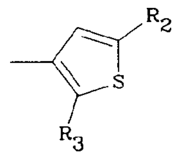 (IB)

dans lesquelles $R_2$ et $R_3$ représentent chacun, indépendamment l'un de l'autre, un groupe alkyle en $C_1$-$C_4$ droit ou ramifié, à l'état de base libre ou de sel d'addition à un acide.

**2.** Composé selon la revendication 1, caractérisé en ce que $R_1$ représente un groupe méthylamino.

**3.** Procédé de préparation d'un composé selon la revendication 1, caractérisé en ce qu'on fait réagir un 2-thiénylimidazo[2,1-*b*]benzothiazole de formule générale (II)

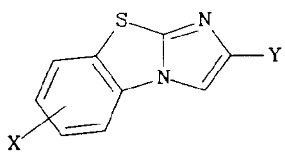

(II)

dans laquelle X et Y sont tels que définis dans la revendication 1,
avec le *N,N*-diméthylglyoxamide, dans un solvant protique à une température de 20 à 80°C, puis on traite le dérivé d'α-hydroxyacétamide de formule générale (III) ainsi obtenu

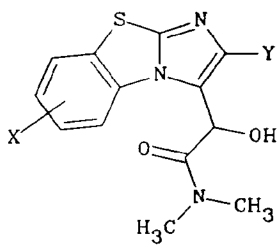

(III)

avec un polyhalogénure d'acide sulfurique ou phosphorique, dans un solvant inerte à une température de 20 à 80°C, pour former le dérivé de α-halogénoacétamide correspondant, puis on fait réagir ce dernier soit avec un agent réducteur tel qu'un hydrure alcalin simple ou complexe, dans un solvant protique, ou dans un solvant inerte miscible à l'eau, à une température de -40 à 40°C, soit avec un agent réducteur tel qu'un hyposulfite ou un dithionite alcalin, ou encore avec l'hydroxyméthylsulfoxylate de sodium, dans un solvant inerte, éventuellement en présence d'un cosolvant inerte miscible à l'eau, à une température de 20 à 40°C, obtenant ainsi le dérivé de *N,N*-diméthyl-2-thiénylimidazo[2,1-*b*]benzothiazole-3-acétamide de formule générale (IV)

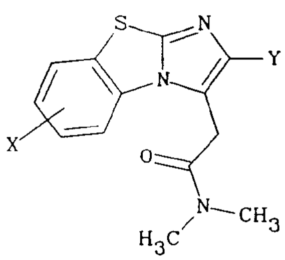

(IV)

qui correspond à la formule générale (I) lorsque R représente un groupe de formule $-N(CH_3)_2$, puis, si on le désire, on transforme ce composé en l'acide de formule générale (I) dans laquelle R représente un groupe hydroxy, par hydrolyse au moyen d'une base forte, et,

a) si on désire préparer un composé de formule générale (I) dans laquelle R représente un groupe de formule générale $-NR_4R_5$ autre qu'un groupe de formule $-N(CH_3)_2$, on fait réagir l'acide de formule générale (I, $R_1$ = OH) avec le *N,N'*-carbonyldiimidazole, dans un solvant inerte, à une température de 20

à 50°C, pour obtenir l'imidazolide correspondant, et finalement on traite ce dernier avec une amine de formule générale $HNR_4R_5$ (dans laquelle $R_4$ et $R_5$ sont tels que définis dans la revendication 1) à une température de 0 à 25°C, ou bien
b) si on désire préparer un composé de formule générale (I) dans laquelle $R_1$ représente un groupe alcoxy, on estérifie l'acide de formule générale (I, $R_1$ = OH).

**4.** Médicament caractérisé en ce qu'il consiste en un composé selon la revendication 1 ou 2.

**5.** Composition pharmaceutique caractérisée en ce qu'elle contient un composé selon la revendication 1 ou 2, associé à un excipient.

Office européen des brevets

# RAPPORT DE RECHERCHE EUROPEENNE

Numero de la demande
EP 93 40 3137

| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (Int.Cl.5) |
| A | CHEMICAL AND PHARMACEUTICAL BULLETIN. vol. 36, no. 12 , 1988 , TOKYO JP pages 4760 - 4768 A. N. EL SHORBAGI ET AL 'Imidazo(2,1-b)thiazoles' * tableau I * --- | 1,4 | C07D513/04<br>A61K31/425<br>//(C07D513/04, 277:00,235:00) |
| A | EP-A-0 313 458 (ROUSSEL-UCLAF) * revendications 1,12 * --- | 1,4 | |
| P,A | EP-A-0 524 055 (SYNTHELABO) * revendications 1,5 * ----- | 1,4 | |
| | | | DOMAINES TECHNIQUES RECHERCHES (Int.Cl.5) |
| | | | C07D<br>A61K |

Le présent rapport a été établi pour toutes les revendications

| Lieu de la recherche | Date d'achèvement de la recherche | Examinateur |
|---|---|---|
| LA HAYE | 18 Avril 1994 | Voyiazoglou, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)